# EUROPEAN PATENT SPECIFICATION

(11) **EP 4 247 331 B1**
(45) Date of publication and mention of the grant of the patent: **08.10.2025**
(21) Application number: 21783588.3
(22) Date of filing: 22.09.2021
(51) Int. Cl.: A61K 9/00, A61K 9/20, A61K 47/12, A61K 47/26, A61K 31/4515, A61K 31/454, A61K 31/4545, A61K 31/495, A61P 37/08

(54) **ORODISPERSIBLE POWDER COMPOSITION COMPRISING AN ANTIHISTAMINE COMPOUND**
IM MUND DISPERGIERBARE PULVERZUSAMMENSETZUNG MIT EINER ANTIHISTAMINVERBINDUNG
COMPOSITION DE POUDRE ORODISPERSIBLE COMPRENANT UN COMPOSÉ ANTIHISTAMINIQUE

(30) Priority: 18.11.2020 EP 20383000
(43) Date of publication of application: 27.09.2023
(73) Proprietor: BioPharma Synergies, S. L., 08028 Barcelona (ES)
(72) Inventor: AGELL PUIG, Francesc, 08028 Barcelona (ES)
(74) Representative: Araujo, Daniel
(86) International application number: PCT/IB2021/058632
(87) International publication number: WO 2022/106923

(56) References cited:
- EP-B1- 2 618 812
- WO-A1-2015/144830
- US-A1- 2015 306 111
- "Germany Approves Antihistamines Xyzal and xusal (levocetirizine)", INTERNET CITATION, 16 January 2001 (2001-01-16), XP002240084, Retrieved from the Internet <URL:http://www.pslgroup.com/dg/1efc66.htmhttp://www.pslgroup.com/dg/1efc66.htm> [retrieved on 20030502]
- ANONYMOUS: "Fever 'n Flu Night Time Sachets - Pharaoina Pharmaceuticals", 13 November 2018 (2018-11-13), XP055874558, Retrieved from the Internet <URL:https://web.archive.org/web/20181113065105/http://pharaoniapharma.com/eneg/product/fever-n-flu-night-time-sachets/> [retrieved on 20211220]
- JIJO ABRAHAM ET AL: "TASTE MASKING OF PEADIATRIC FORMULATION: A REVIEW ON TECHNOLOGIES, RECENT TRENDS AND REGULATORY ASPECTS", INTERNATIONAL JOURNAL OF PHARMACY AND PHARMACEUTICAL SCIENCES, vol. 6, no. 1, 1 January 2014 (2014-01-01), pages 12 - 19, XP055503411

## Description

### Technical Field

The present invention relates to pharmaceutical compositions comprising an antihistamine compound as active ingredient, which can be dosed in the form of stick packs as orodispersible powders or formulated as tablets.

### Background art

Histamine is a physiologically active, endogenous substance that is derived from the decarboxylation of the amino acid histidine. In humans, histamine exerts its effects primarily by binding to G protein-coupled histamine receptors, designated H₁ through H₄, which become apparent as direct and indirect microvascular dilation and increased vascular permeability, as well as activation of specific receptors in the nose, eyes, respiratory tract, and skin, causing characteristic allergic signs and symptoms.

Antihistamine compounds are compounds that antagonize histamine at receptor sites, which are often used to relieve symptoms of allergies, such as hay fever, hives, conjunctivitis, and reactions to insect bites or stings.

The oral route of administration is the preferred administration route for patients suffering allergic reactions.

Orodispersible formulations are an alternative for patients who prefer not to take conventional tablets, for patients who have nausea and vomiting, and for patients with dysphagia.

A common problem associated with pharmaceutical formulations of pharmaceutical active ingredient is the unpleasant bitter taste they present, as disclosed in US-A-2006/198885.

In the state of the art, different approaches to mask the bitterness of antihistamine actives have been disclosed: use of complexes with ion exchange resins, combination with cyclodextrin, use of polymer coating, use of specific taste-masking compounds, or use of effervescent compositions.

For example, in WO-A-00/18365 it is disclosed a fast dissolving orally consumable films, wherein the active ingredient, e.g. loratadine may be coated to mask the taste. The coating of the active ingredient is also proposed in WO-A-2004/066925, wherein the coating is selected from ethylcellulose, hydroxyethylcellulose, hydroxypropylmethylcellulose, hydroxypropylmethyl cellulose phthalate, methacrylic copolymer, lipidic compounds, lipophilic compounds, polyethylene glycol behenate, glycerol palmitostearate, glycerol stearate, cetyl palmitate, glyceric macrogol beeswaxes, glycerol, PEG-32 stearate, Peg-32 palmitostearate, and mixtures thereof. An analogous solution is proposed in WO-A-2008/019854 disclosing a pharmaceutical composition for oral administration comprising at least one particle containing an active ingredient, e.g. loratadine, said particle comprising: a) a core which contains the active ingredient of the particle and, optionally, suitable adjuvants, and b) more coating layers applied to the core, when more than one coating layer is present, contains: (i) at least one polymer capable of forming a gel with water, (ii) at least one polymer not capable of forming a gel with water, (iii) at least one compound capable of releasing carbon dioxide, and (iv) optionally, additional adjuvants. A hot-melt coating of fexofenadine is disclosed in WO-A-2011/132167 allowing an efficient masking of the bitter taste of the active. Though the coating may mask the unpleasant taste, if the thickness and composition of the coating is not properly controlled, it may affect the disintegration and dissolution characteristics of the tablet. Further, the coating operation is a highly controlled and costly process.

In WO-A-01/70194 it is proposed the use of an ion exchange resin to mask the taste of the active ingredient, e.g. loratadine, present in a fast dissolving orally consumable film. A resinate comprising desloratadine complexes with a cation-exchange resin is disclosed in IN-A-02796DE2005. In CN-A-104306338 it is disclosed a clathrate compound between desloratadine and an acrylic acid resin, which improves significantly the taste.

In WO-A-02/074238 it is disclosed a composition having a pharmaceutically acceptable carrier and an active agent (e.g. loratadine) complexed with glycyrrhizin.

In CN-A-1031127012 it is disclosed a tablet comprising a hot-melt solid dispersion of ebastine in a binder to improve the taste of the active.

In WO-A-99/01133 and in WO-A-2010/028101 it is disclosed a tablet comprising cetirizine, wherein said active is in the form of a complex with cyclodextrin. Cyclodextrin is also used as taste-masking agent for levocetirizine in the composition disclosed in WO-A-2015/144830. In WO-A-2011/110939 a polyol is further included to the cyclodextrin inclusion complex of an active ingredient, e.g. cetirizine, or levocetirizine, among others, to masking the bitter taste thereof.

In WO-A-2006/058022 it is disclosed an antihistamine composition comprising a taste-masking agent selected from the group consisting of sucralose, sucrose, saccharin or a salt thereof, fructose, dextrose, corn syrup, aspartame, acesulfame-K, xylitol, sorbitol, erythritol, ammonium glycyrrhizinate, thaumatin, neotame, mannitol, menthol, eucalyptus oil, camphor, a natural flavouring agent, an artificial flavouring agent, and combinations thereof.

In WO-A-2011/146030 it is disclosed the use of effervescent pharmaceutical compositions to mask the bitter taste of desloratadine. Analogously, in WO-A-11/146032 it is disclosed an effervescent formulation comprising levocetirizine.

In US-A-2013/0217697 it is disclosed a taste-masking agent for cetirizine, which comprises one or more inorganic and/or organic salt(s) and at least two monocyclic monoterpenes as well as one or more sweetener(s).

In WO-A-2013/058496 the taste-masked formulation is prepared by wet granulation of a mixture comprising the pharmaceutically active ingredient (e.g. cetirizine, among others), at least one compound selected from the group consisting of magnesium aluminometasilicate and calcium silicate, and a pharmaceutically acceptable carrier.

In WO-A-2019/219143 it is disclosed an oral tablet for taste masking of active ingredients, the tablet comprising a population of particles and an active ingredient with off-note taste (e.g. cetirizine, loratadine or desloratadine, among others), the population of particles comprising directly compressible (DC) and non-directly compressible (non- DC) sugar alcohol particles, the non-DC particles providing the tablet with a plurality of discrete non-DC areas, and the non-DC areas resulting at least partly in induced taste masking of the active ingredient upon mastication of the tablet.

A different approach is disclosed in WO-A-2004/019885, wherein a compound comprising a purine or pyrimidine group or derivative thereof bonded to a ribose or deoxyribose sugar moiety, or a derivative of ribose or deoxyribose and an ionizable phosphate is used to decrease or abrogate the perception of bitterness of bitter active ingredients.

Another approach is disclosed in WO-A-2019/150388, wherein fructo-oligosaccharide and glucose oxidase are used for masking undesirable taste of certain substances and products consumed or administered orally, such as, e.g. loratadine, desloratadine or cetirizine, among other compounds.

In IN-A-00674DE1999 it is disclosed that the taste masking of cetirizine may be carried out by coating of drug particles, embedment of the drug particles in an inert matrix, or complexation.

Despite the various proposals available in the state of the art, there is still a need to have new oral pharmaceutical compositions, comprising an antihistamine active compound, having a masked taste and a high degree of acceptance by the patient.

### Object of the invention

The object of the present invention is an orodispersible powder composition as defined in claim 1.

A stick pack comprising the orodispersible powder composition is also part of the invention.

A tablet comprising the orodispersible powder composition is also part of the invention.

The orodispersible powder composition, stick pack, or tablet for use in the treatment of allergic diseases is also part of the invention.

### Description of the Drawings

Figure 1 represents a mixture diagram to test ten specific combinations of sodium citrate, citric acid and sucralose, to obtain an equation to describe the behaviour of the ternary system. In that diagram, X1 represents sodium citrate, X2 citric acid and X3 sucralose.
Figure 2 represents the isolines corresponding to the degree of acceptance for the orodispersible powders containing bilastine as active pharmaceutical ingredient, and the synergistic ternary combination of sodium citrate (X1), citric acid (X2) and sucralose (X3).
Figure 3 represents the isolines corresponding to the degree of acceptance for the orodispersible powders containing levocetirizine as active pharmaceutical ingredient, and the synergistic ternary combination of sodium citrate (X1), citric acid (X2) and sucralose (X3).

### Detailed description of the invention

The object of the present invention is an orodispersible powder composition, which comprises:
a) a pharmacologically effective amount of an antihistamine compound or a pharmaceutically acceptable salt thereof,
b) a ternary combination consisting of sodium citrate, citric acid and sucralose, and
c) one or more further excipients,

wherein the ratio a):b) is comprised from 1:2 to 1:75,
wherein the ternary composition comprises 55% to 75% by weight of sodium citrate, 15% to 35% by weight of citric acid and 10% to 30% by weight of sucralose, wherein the amounts of sodium citrate, citric acid and sucralose in the ternary composition are combined to make 100%,
and wherein the content of the ternary combination in the orodispersible powder composition is comprised between 5% and 20% by weight over the total weight of the orodispersible powder composition.

In an embodiment, the orodispersible powder composition is not granulated.

In an embodiment, the orodispersible powder composition is not effervescent. In a preferred embodiment, the orodispersible powder composition is not effervescent.

The authors of the present invention have developed an orodispersible powder by direct mixing with optimum flow properties, masking the bitter taste of antihistamine active compounds, and exhibiting an excellent acceptance of the formulation by the patient. The masking of the bitter taste has been surprisingly produced by a synergistic effect between citric acid, sodium citrate and sucralose. The orodispersible formulation dissolves rapidly in the oral cavity (less than 15 seconds), leaving no product residue in the mouth, and without the need to drink water after administration. Additionally, orodispersible powders of the invention show surprisingly optimal flow properties even after long periods of storage at room temperature.

The orodispersible powder of the invention allows improving the treatment compliance in patients with allergy and reducing manufacturing costs. Further, the formulation is suitable for diabetics and for lactose intolerant.

In the context of the present invention, an orodispersible powder is a pharmaceutical composition, wherein the active pharmaceutical ingredient and the excipients are in powder form and which disperses and/or dissolves rapidly in the oral cavity. Rapidly means in less than 30 seconds, preferably in less than 20 seconds, and more preferably in less than 15 seconds.

In the context of the present invention, the term "antihistamine active compound" refers both to an antihistamine active compound and to a pharmaceutically acceptable salt thereof.

In the present description, as well as in the claims, the singular forms "a", "an" and "the" include the plural reference unless the context clearly indicates otherwise. The ranges defined by the preposition the terms "between ... and ..." or by the terms "from ...to..." include also the two ends thereof. The term "about" refers to a deviation of plus/minus 10%, preferably plus/minus 5%. The percentages are expressed in % by weight, unless stated the contrary.

### Antihistamine active compound

The active ingredient of the orodispersible powder of the present invention is an antihistamine active compound or a pharmaceutically acceptable salt thereof.

As used herein, the term "pharmaceutically acceptable salt" refers to a salt prepared from pharmacologically acceptable anions, such as, for example, acetate, adipate, anthranilate, ascorbate, benzenesulfonate, benzoate, bisulfate, bitartrate, camphorsulfonate, ethanesulfonate, formate, fumarate, furoate, galacturonate, gentisate, gluconate, glucuronate, glutamate, glycolate, hydrobromide, hydrochloride, hydroiodide, isethionate, isonicotinate, lactate, malate, maleate, malonate, mandelate, mesylate, mucate, nitrate, pamoate, pantothenate, phentylacetate, phosphate, propionate, saccharate, salicylate, stearate, succinate, sulfanilate, sulfate, tartrate, *p-*toluenesulfonate, and hydrates thereof. Preferably the pharmaceutically acceptable salt is selected from hydrochloride, fumarate and hydrates thereof.

Antihistamine active compounds are pharmaceutical active ingredients which are used in the treatment of allergy, such as, for example, antihistamines such as acrivastine, azatadine, azelastine, bilastine, brompheniramine, carbinoxamine, cetirizine, chlorpheniramine, clemastine, desloratadine, dexbropheniramine, dexchlorpheniramine, diphenylhydramine, diphenylpyraline, doxylamine, ebastine, fexofenadine, levocetirizine, loratadine, olopatadine, promethazine, pyrilamine, rupatadine, tripelennamine, triprolidine, and the like

The antihistamine active compound is preferably selected from acrivastine, azatadine, azelastine, bilastine, brompheniramine, carbinoxamine, cetirizine, chlorpheniramine, clemastine, desloratadine, dexbropheniramine, dexchlorpheniramine, diphenylhydramine, diphenylpyraline, doxylamine, ebastine, fexofenadine, levocetirizine, loratadine, olopatadine, promethazine, pyrilamine, rupatadine, tripelennamine, triprolidine, or a salt thereof, and mixtures thereof; more preferably selected from desloratadine, loratadine, cetirizine, levocetirizine, rupatadine, bilastine, ebastine, or a salt thereof, and mixtures thereof.

In an embodiment, the antihistamine active compound is preferably selected from the group of desloratadine and loratadine or a salt thereof.

In another embodiment, the antihistamine active compound is preferably selected from the group of cetirizine and levocetirizine or a salt thereof.

In another embodiment, the antihistamine active compound is rupatadine or a salt thereof.

In another embodiment, the antihistamine active compound is bilastine or a salt thereof.

In another embodiment, the antihistamine active compound is ebastine or a salt thereof.

The pharmacologically effective amount of each antihistamine active compound is well-known by the skilled person in the art. In commercially available medicinal products in the form of tablets, recommended doses are, for example: 5 mg for levocetirizine dihydrochloride and desloratadine, 10 mg for rupatadine, cetirizine dihydrochloride and loratadine, and 10 mg or 20 mg for ebastine and bilastine.

Antihistamine active compounds and pharmaceutically acceptable salts thereof are available active pharmaceutical ingredients, either commercially, for example, through the company Sigma-Aldrich (ebastine, bilastine, rupatadine, cetirizine, levocetirizine, loratadine, desloratadine), or they can be prepared according to processes already disclosed in prior art, for example, EP-A-134124 (ebastine), bilastine (EP-A-0818454), rupatadine (ES-A-2042421), cetirizine (EP-A-0058146), loratadine (US4282233).

The content of the antihistamine active compound in the orodispersible powder of the invention is usually comprised from 0.05% to 10%, preferably from 0.1% to 5% by weight over the total weight of the orodispersible powder composition. If a pharmaceutically acceptable salt of an antihistamine active compound is used in the formulation, then the amount is corrected accordingly.

### Ternary combination

The orodispersible powder of the invention comprises a synergistic ternary combination consisting of sodium citrate, citric acid and sucralose. Preferably a ternary combination of sodium citrate anhydrous, citric acid anhydrous and sucralose.

The synergistic ternary combination is formulated in the orodispersible powder of the invention in a ratio antihistaminic active compound:ternary combination comprised between 1:2 and 1:75, preferably between 1:5 and 1:75, preferably between 1:6 to 1:70, more preferably between 1:7 and 1:65, and yet more preferably between 1:12.5 and 1:60. In a preferred embodiment the ratio is selected from the group consisting of about 1:12.5, about 1:25, about 1:30, about 1:50 and about 1:60.

Sodium citrate, as either the dihydrate or anhydrous material, is used in pharmaceutical formulations. It is used primarily as a buffering agent to adjust the pH.

Citric acid as either the monohydrate or anhydrous material, is used in pharmaceutical formulations. It is used primarily as a buffering agent to adjust the pH.

In the composition of the invention, sodium citrate is preferably anhydrous and citric acid is preferably anhydrous.

Sucralose is used as a sweetening agent in pharmaceutical applications. It has a sweetening power approximately 300-1000 times that of sucrose and has no aftertaste. It has no nutritional value, is noncariogenic, does not promote dental caries, and produces no glycemic response.

In the ternary combination, the content of sodium citrate is comprised from 55% to 75%, and more preferably from 60% to 70% by weight over the total weight of the ternary composition.

In the ternary combination, the content of citric acid is comprised from 15% to 35%, and more preferably from 20% to 30% by weight over the total weight of the ternary composition.

In the ternary combination, the content of sucralose is comprised from 10% to 30%, and more preferably from 15% to 25% by weight over the total weight of the ternary composition.

The amounts of sodium citrate, citric acid and sucralose in the ternary composition are combined to make 100%. If hydrated forms of sodium citrate and/or citric acid are used, the amounts are corrected accordingly.

Preferred embodiments of the ternary composition comprise 55% to 75% by weight of sodium citrate, 15% to 35% by weight of citric acid and 10% to 30% by weight of sucralose, more preferably 58% to 70% by weight of sodium citrate, 18% to 30% by weight of citric acid and 13% to 25% by weight of sucralose, and more preferably 60% to 65% by weight of sodium citrate, 20% to 25% by weight of citric acid and 15% to 20% by weight of sucralose, wherein the amounts of sodium citrate, citric acid and sucralose in the ternary composition are combined to make 100%.

The content of the ternary combination in the orodispersible powder composition is comprised between 5% and 20%, preferably between 7% and 18%, more preferably between 10% and 15%, and yet more preferably between 11% and 12% by weight over the total weight of the orodispersible powder composition.

Surprisingly, the ternary combination is able to mask the bitter taste of antihistamine active compounds a long of a broad range of ratios between the antihistamine active compound and ternary combination, between 1:2 and 1:75, preferably between 1:5 and 1:75, preferably between 1:6 to 1:70, more preferably between 1:7 and 1:65, and yet more preferably between 1:12.5 and 1:60. As shown in the examples, a ratio antihistamine active compound:ternary combination of about 1:12.5 was exemplified for bilastine and ebastine, about 1:25 for loratadine and rupatadine, about 1:30 for cetirizine, about 1:50 for desloratadine and about 1:60 for levocetirizine.

### Other excipients

The orodispersible powder of the invention may comprise generally further excipients, such as diluents, lubricants, glidants, flavouring agents, sweetening agents, acidulants, alkalizing agents, buffering agents, antioxidants and mixtures thereof.

Suitable excipients for use in the preparation of the orodispersible powder of the invention are described, for example, in the book Handbook of pharmaceutical excipients, R.C. Rowe, P.J. Sheskey and M.E. Quinn, editors, Pharmaceutical Press, sixth edition, 2009.

Further excipients that are usually included in the orodispersible powder of the invention are selected from the group comprising diluents, lubricants, glidants, flavouring agents and mixtures thereof.

In a preferred embodiment, the orodispersible powder comprise one or more further excipients selected from diluents, lubricants, glidants, flavouring agents and mixtures thereof.

Suitable diluents may comprise, for example, mannitol, sorbitol, xylitol, isomalt, erythritol, cellulose powdered, microcrystalline cellulose, silified microcrystalline cellulose, starch, calcium phosphate, calcium lactate, calcium carbonate, magnesium carbonate, magnesium oxide and mixtures thereof; preferably the diluent is mannitol. In a preferred embodiment, the composition does not comprise lactose.

Mannitol is used in pharmaceutical formulations. It is primarily used as a diluent, where it is of particular value since it is not hygroscopic and may thus be used with moisture-sensitive active ingredients. It is commonly used as an excipient in the manufacture of chewable tablet formulations because of its negative heat of solution, sweetness, and 'mouth feel', imparting a cooling sensation in the mouth. It is also used as a diluent in rapidly dispersing oral dosage forms. Given orally, mannitol is not absorbed significantly from the gastrointestinal tract, and it is suitable for diabetic patients. Mannitol does not undergo Maillard reactions. It has a low caloric content and it is noncariogenic.

Generally, the diluent is present in an amount from 65% to 95%, preferably from 75% to 90%, more preferably from 80% to 89%, and yet more preferably from 85% to 88% by weight over the total weight of the orodispersible powder composition.

The orodispersible powder may further comprise a lubricant, wherein the lubricant is usually selected from the group consisting of talc, sodium benzoate, sodium stearyl fumarate, sodium lauryl sulfate, calcium stearate, magnesium stearate, zinc stearate, glyceryl behenate, stearic acid, glyceryl monostearate, poloxamer, polyethylene glycol and mixtures thereof; preferably the lubricant is sodium stearyl fumarate.

Sodium stearyl fumarate is used as a lubricant in pharmaceutical compositions. It is supplied in a pure form and is of value when the less pure stearate-type lubricants are unsuitable owing to chemical incompatibility. Sodium stearyl fumarate is less hydrophobic than magnesium stearate or stearic acid and has a less retardant effect on dissolution than magnesium stearate.

Generally, the lubricant is present in an amount from 0.05% to 10%, preferably from 0.1% to 5%, more preferably from 0.2% to 2.5%, more preferably from 0.4% to 1%, more preferably from 0.45% to 0.75%, and yet more preferably about 0.5% by weight over the total weight of the orodispersible powder composition.

The orodispersible powder may further comprise a glidant, wherein the glidant is usually selected from the group consisting of colloidal silicon dioxide, calcium phosphate tribasic, hydrophobic colloidal silica, magnesium silicate, magnesium trisilicate, silicon dioxide, talc and mixtures thereof. The preferred glidant is colloidal silicon dioxide.

Colloidal silicon dioxide is used in pharmaceutical formulations. Its small particle size and large specific surface area give it desirable flow characteristics that are exploited to improve the flow properties of dry powders.

Generally, the glidant is present in an amount from 0.05% to 10%, preferably from 0.1% to 5%, more preferably from 0.2% to 2.5%, more preferably from 0.4% to 1%, more preferably from 0.45% to 0.75%, and yet more preferably about 0.5% by weight over the total weight of the orodispersible powder composition.

The orodispersible powder may further comprise a flavouring agent, wherein it is usually selected from fruit flavours such as orange, banana, strawberry, cherry, wild cherry, lemon and mixtures thereof, and other flavours such as cardamom, anise, peppermint, menthol, vanillin and ethyl vanillin and mixtures thereof. preferably the flavouring agent comprises peppermint flavour.

Generally, the content of the flavouring agent is comprised from 0.05% to 5.0% by weight over the total weight of the orodispersible powder composition.

In some embodiments, the orodispersible powder may comprise a sweetening agent, which is usually selected from the group consisting of saccharin sodium, neohesperidin dihydrochalcone, acesulfame potassium, aspartame and mixtures thereof. Preferably the composition does not include sucrose, and therefore it is suitable for diabetics.

Generally, the sweetening agent is usually present in an amount from 0.1% to 5% by weight over the total weight of the orodispersible powder composition.

In some embodiments, the orodispersible powder may comprise an acidulant, which is usually selected from the group consisting of fumaric acid, maleic acid, malic acid, tartaric acid and mixtures thereof.

Generally, the content of the acidulant is comprised from 0.1% to 2.0% by weight over the total weight of the orodispersible powder composition.

In some embodiments, the orodispersible powder may comprise an alkalizing agent, which is usually selected from the group consisting of potassium bicarbonate, potassium citrate, sodium bicarbonate, sodium carbonate and mixtures thereof. Generally, the alkalizing agent is usually present in an amount from 1% to 40% by weight over the total weight of the orodispersible powder composition.

In some embodiments, the orodispersible powder may comprise a buffering agent, which is usually selected from the group consisting of dibasic sodium phosphate, glycine, methionine and mixtures thereof.

Generally, the content of the buffering agent is comprised from 1% to 40% by weight over the total weight of the orodispersible powder composition.

In some embodiments, the orodispersible powder may comprise an antioxidant, which is usually selected from the group consisting of ascorbic acid, erythorbic acid, sodium ascorbate and mixtures thereof.

Generally, the antioxidant is usually present in an amount from 0.1% to 5% by weight over the total weight of the orodispersible powder composition.

### Manufacturing process

The process for preparing the orodispersible powder is a direct mixing of the components, which makes very attractive for industrial manufacturing. The process comprises generally sieving of the components through a 0.5 mm - 1 mm mesh sieve, and mixing in, for example, a V-shaped mixer, which is suitable for free-flowing powders. Mixing time is adjusted according to routine practice to obtain a homogeneous mixture, which is dosed in stick packs.

Conventional mixing procedures, well-known to the skilled in pharmaceutical technology, are disclosed in reference books in the field, for example, in the book Aulton's Pharmaceutics. The design and manufacture of medicines, M.E. Aulton and K.M.G. Taylor, editors, Churchill Livingstone Elsevier, Fourth Edition, 2013; or in the book Remington: The Science and Practice of Pharmacy, D. Limmer editor, Lippincott Williams & Wilkins, 2000.

### Stick pack

A stick pack comprising the orodispersible powder of the invention also forms part of the invention.

A stick pack is a packaging form, usually having a laminate structure to give a specific barrier and sealing characteristics. It is used for a single dose administration. It is of common use containing pharmaceutical compositions or foods, e.g. sugar. Materials usually employed to manufacture stick packs are aluminium, paper, polyethylene, polyethylene terephthalate, propylene and combinations thereof.

The stick pack comprises a dose of a pharmacologically effective amount of the orodispersible powder composition, which comprises an antihistamine active compound. The stick pack preferably comprises an amount of the orodispersible powder composition that comprises 4.2 mg levocetirizine (equivalent to 5 mg levocetirizine dihydrochloride), 5 mg desloratadine, 10 mg loratadine, 10 mg rupatadine (equivalent to 12.8 mg rupatadine fumarate), 8.4 mg cetirizine (equivalent to 10 mg cetirizine dihydrochloride), 20 mg ebastine or 20 mg bilastine, or a pharmaceutically acceptable salt thereof.

The stick pack usually comprises an amount of the orodispersible powder composition, which is comprised between 1 g and 10 g, preferably between 1.5 g and 5 g, more preferably between 1.9 g and 3 g, and yet more preferably about 2.2 g. Preferably it comprises 4.2 mg levocetirizine (equivalent to 5 mg levocetirizine dihydrochloride), 5 mg desloratadine, 10 mg loratadine, 10 mg rupatadine (equivalent to 12.8 mg rupatadine fumarate), 8.4 mg cetirizine dihydrochloride (equivalent to 10 mg cetirizine dihydrochloride) or 20 mg ebastine or 20 mg bilastine, or a pharmaceutically acceptable salt thereof.

The new orodispersible powder formulation obtained by direct mixing of the components provides an excellent masking of the bitter taste of the antihistamine active compound with an excellent acceptance of the formulation by the patient.

Orodispersible powders that are obtained by direct mixing of antihistamine active compounds, and stick packs comprising the orodispersible powder composition obtained by direct mixing, are not commercially available.

Direct mixing of the components allows the direct exposure of the active ingredient in the oral cavity, increasing the dissolution rate of the active ingredient and its absorption in the oral cavity, favouring the fast effect of the medication, which is considered a very important attribute and valued by both patients and doctors.

In this invention the stick pack packaging form has been chosen because it is practical, robust, easy to transport and use individually, so it adapts well to a modern and active lifestyle. The patient can carry the stick pack always on top and, therefore, if an allergic reaction occurs, the medication can be taken just after the onset of the allergic reaction without the need to drink water, increasing the effectiveness of the antihistamine active compound. The orodispersible powder composition of the invention has industrial application, since a direct mixing process is used as manufacturing process, which is widely used in the pharmaceutical industry, and, in addition, as shown in the examples, the formulation exhibits optimal rheological characteristics according to the European Pharmacopoeia current edition, and, therefore, it can be scale up at industrial level, so that it can be dosed on equipment with the stick pack packaging format.

As shown in the examples, the orodispersible powder composition of the invention shows a high degree of acceptance by the patient for all antihistamine active compounds over a broad range of compositions.

In an embodiment, the orodispersible powder composition can be compressed into a tablet.

### Use

The orodispersible powder composition and the stick pack of the invention for use in the treatment of allergic diseases also forms part of the invention.

In a preferred embodiment, the orodispersible powder composition and the stick pack of the invention are for use in the treatment of an allergic disease selected from seasonal allergic rhinitis, persistent allergic rhinitis, seasonal allergic rhino-conjunctivitis, perennial allergic rhino-conjunctivitis, urticaria, chronic idiopathic urticaria, and allergic dermatitis.

In the following examples, different embodiments of the invention are provided for illustrative purposes that are understood to be non-limiting.

### Examples

### Examples 1-10: Orodispersible powders comprising bilastine

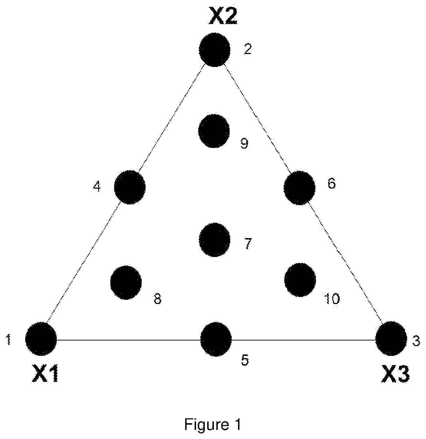

Orodispersible powders comprising bilastine as active ingredient were prepared according to a mixture design (see Figure 1). This methodology is well-known and disclosed, for example, in J. Cornell, Experiments with Mixtures, 3 rd edition, John Wiley & Sons, 2002. The matrix of the design of experiments is shown in Table I.

Examples 1-6 are not according to the claimed invention.

**TABLE I**

| **Example** | **A** | **B** | **C** | **Citrate** | **Citric** | **Sucralose** | **Total** |
|---|---|---|---|---|---|---|---|
| 1 | 1 | 0 | 0 | 85 | 10 | 5 | 100 |
| 2 | 0 | 1 | 0 | 50 | 45 | 5 | 100 |
| 3 | 0 | 0 | 1 | 50 | 10 | 40 | 100 |
| 4 | 1/2 | 1/2 | 0 | 67.5 | 27.5 | 5 | 100 |
| 5 | 1/2 | 0 | 1/2 | 67.5 | 10 | 22.5 | 100 |
| 6 | 0 | 1/2 | 1/2 | 50 | 27.5 | 22.5 | 100 |
| 7 | 1/3 | 1/3 | 1/3 | 61.6 | 21.7 | 16.7 | 100 |
| 8 | 2/3 | 1/6 | 1/6 | 73.3 | 15.8 | 10.9 | 100 |
| 9 | 1/6 | 2/3 | 1/6 | 55.8 | 33.3 | 10.9 | 100 |
| 10 | 1/6 | 1/6 | 2/3 | 55.8 | 15.8 | 28.4 | 100 |

The specific composition of the ternary combination of these powders, expressed in mg, is shown in Table II:

**TABLE II**

| **Component** | **Ex. 1** | **Ex. 2** | **Ex. 3** | **Ex. 4** | **Ex. 5** |
|---|---|---|---|---|---|
| Sodium citrate anh. | 212.5 | 125.0 | 125.0 | 168.8 | 168.8 |
| Citric acid anh. | 25.0 | 112.5 | 25.0 | 68.7 | 25.0 |
| Sucralose | 12.5 | 12.5 | 100.0 | 12.5 | 56.2 |

**TABLE II (cont)**

| **Component** | **Ex. 6** | **Ex. 7** | **Ex. 8** | **Ex. 9** | **Ex. 10** |
|---|---|---|---|---|---|
| Sodium citrate anh. | 125.0 | 154.0 | 183.3 | 139.5 | 139.5 |
| Citric acid anh. | 68.7 | 54.3 | 39.5 | 83.3 | 39.5 |
| Sucralose | 56.3 | 41.7 | 27.3 | 27.3 | 71.0 |

In addition to the ternary composition, each composition contained the following components in mg, as shown in Table III:

**TABLE III**

| **Component** | **mg** |
|---|---|
| Bilastine | 20 |
| Mannitol | 1903 |
| Sodium stearyl fumarate | 11 |
| Colloidal silicon dioxide | 11 |
| Flavouring agent | 5 |

In all these examples, the total amount of the composition is 2200 mg, and the content of the ternary combination in the composition is 11.36 wt.%

In these examples the ratio active ingredient:ternary combination was 1:12.5, i.e. 20 mg of bilastine and 250 mg of the ternary combination.

The process for preparing the orodispersible powders comprised the following steps:
1) Sieving the components through a 0.5 mm mesh sieve (except the citric acid anhydrous that is sieved through a 1 mm mesh sieve), and incorporate them into the V-shaped mixer in the following order: sodium citrate anhydrous, sucralose, flavouring agent, active ingredient (bilastine), colloidal silicon dioxide, mannitol, and citric acid anhydrous.
2) Mixing about 15 minutes at about 20 rpm in the V-mixer.
3) Sieving the sodium stearyl fumarate through a 0.5 mm mesh sieve and incorporate it into the V-mixer.
4) Mix about 5 minutes at about 20 rpm in the V-mixer.
5) Dosing the product in stick packs (2200 mg per stick).

It was also confirmed that powders dissolve rapidly in the oral cavity, leaving no product residue in the mouth.

### Examples 11-20: Orodispersible powders comprising levocetirizine

Orodispersible powders comprising levocetirizine as active ingredient were prepared according to a mixture design (see Figure 1) and according to the matrix shown above in Table I.

The specific compositions of the ternary combination of Examples 11 to 20, expressed in mg, are the same as shown in Table II above for Examples 1 to 10.

### Examples 11-16 are not according to the claimed invention.

In addition to the ternary composition, each composition contained the following components in mg, as shown in Table IV:

**TABLE IV**

| **Component** | **mg** |
|---|---|
| Levocetirizine dihydrochloride | 5 |
| Mannitol | 1918 |
| Sodium stearyl fumarate | 11 |
| Colloidal silicon dioxide | 11 |
| Flavouring agent | 5 |

In all these examples, the total amount of the composition is 2200 mg, and the content of the ternary combination in the composition is 11.36 wt.%

In these examples the ratio active ingredient:ternary combination was 1:60, i.e. 4.2 mg of levocetirizine (corresponding to 5 mg of levocetirizine dihydrochloride) and 250 mg of the ternary combination.

The process for preparing the orodispersible powders is analogous to the process disclosed above in Examples 1 to 10.

It was also confirmed that powders dissolve rapidly in the oral cavity, leaving no product residue in the mouth.

### Examples 21 and 22: Orodispersible powder comprising loratadine or cetirizine

Following an analogous process disclosed above in Examples 1 to 10, an orodispersible powder comprising loratadine or cetirizine as active ingredient was prepared according to the composition, expressed in mg, of Table VI:

**TABLE VI**

| **Component** | **Ex. 21** | **Ex.22** |
|---|---|---|
| Loratadine | 10 | - |
| Cetirizine dihydrochloride | - | 10 |
| Sodium citrate anh. | 154.0 | |
| Citric acid anh. | 54.3 | |
| Sucralose | 41.7 | |
| Mannitol | 1913 | |
| Sodium stearyl fumarate | 11 | |
| Colloidal silicon dioxide | 11 | |
| Flavouring agent | 5 | |

In this example the ratio loratadine:ternary combination was 1:25, i.e. 10 mg of active compound and 250 mg of the synergistic combination, and the ratio cetirizine:ternary combination was 1:30, i.e. 8.4 mg of cetirizine (corresponding to 10 mg of cetirizine dihydrochloride) and 250 mg of the ternary combination; and the total amount of the composition is 2200 mg, wherein the content of the ternary combination in the composition is 11.36 wt.%

It was also confirmed that the powder dissolves rapidly in the oral cavity, leaving no product residue in the mouth.

### Example 23: Orodispersible powder comprising rupatadine

Following an analogous process disclosed above in Examples 1 to 10, an orodispersible powder comprising rupatadine as active ingredient was prepared according to the composition, expressed in mg, of Table VII:

**TABLE VII**

| **Component** | **Ex. 21** |
|---|---|
| Rupatadine fumarate | 12.8 |
| Sodium citrate anh. | 154.0 |
| Citric acid anh. | 54.3 |
| Sucralose | 41.7 |
| Mannitol | 1910.2 |
| Sodium stearyl fumarate | 11 |
| Colloidal silicon dioxide | 11 |
| Flavouring agent | 5 |

In this example the ratio active ingredient:ternary combination was 1:25, i.e. 10 mg of rupatadine (corresponding to 12.8 mg of rupatadine fumarate) and 250 mg of the synergistic combination, and the total amount of the composition is 2200 mg, wherein the content of the ternary combination in the composition is 11.36 wt.%

It was also confirmed that the powder dissolves rapidly in the oral cavity, leaving no product residue in the mouth.

### Example 24: Orodispersible powder comprising desloratadine

Following an analogous process disclosed above in Examples 1 to 10, an orodispersible powder comprising desloratadine as active ingredient was prepared according to the composition, expressed in mg, of Table VIII:

**TABLE VIII**

| **Component** | **Ex. 24** |
|---|---|
| Desloratadine | 5 |
| Sodium citrate anh. | 154.0 |
| Citric acid anh. | 54.3 |
| Sucralose | 41.7 |
| Mannitol | 1918 |
| Sodium stearyl fumarate | 11 |
| Colloidal silicon dioxide | 11 |
| Flavouring agent | 5 |

In this example the ratio active ingredient:ternary combination was 1:50, i.e. 5 mg of desloratadine and 250 mg of the synergistic combination, and the total amount of the composition is 2200 mg, wherein the content of the ternary combination in the composition is 11.36 wt.%

It was also confirmed that the powder dissolves rapidly in the oral cavity, leaving no product residue in the mouth.

### Example 25: Orodispersible powder comprising ebastine

Following an analogous process disclosed above in Examples 1 to 10, an orodispersible powder comprising ebastine as active ingredient was prepared according to the composition, expressed in mg, of Table IX:

**TABLE IX**

| **Component** | **Ex. 25** |
|---|---|
| Ebastine | 20 |
| Sodium citrate anh. | 154.0 |
| Citric acid anh. | 54.3 |
| Sucralose | 41.7 |
| Mannitol | 1903 |
| Sodium stearyl fumarate | 11 |
| Colloidal silicon dioxide | 11 |
| Flavouring agent | 5 |

In this example the ratio active ingredient:ternary combination was 1: 12.5, i.e. 20 mg of ebastine and 250 mg of the synergistic combination, and the total amount of the composition is 2200 mg, wherein the content of the ternary combination in the composition is 11.36 wt.%

It was also confirmed that the powder dissolves rapidly in the oral cavity, leaving no product residue in the mouth.

### Example 26: Testing of orodispersible powders comprising antihistamine compounds

### a) Organoleptic testing

Organoleptic tests of the orodispersible powder formulations dosed in stick packs, were carried out in 5 healthy volunteers.

The methodology for evaluating the acceptance of the formulations by the patient has consisted in establishing a score from 1 to 5 for the taste of the formulation (acid, sweet or salty), where 1 corresponds to an unacceptable taste, and 5 corresponds to an excellent taste, and another score from 1 to 5 to assess the bitterness of the formulation, wherein 1 corresponds to an unacceptable bitterness, and 5 corresponds to an excellent capacity for masking the bitterness of the active ingredient. The multiplication of the taste value of the formulation by the value referring to the capacity of masking the bitterness of the active ingredient, gives rise to a value that is the degree of acceptance of the formulation by the patient.

The results obtained indicate that the formulations mask the bitter taste of the antihistamine active compounds, with an excellent acceptance of the formulation by the patient, and without the need to drink water after the administration of the orodispersible formulation. Table X shows the results of several orodispersible powder formulations according to the invention:

**TABLE X**

| **Example** | **Bitterness** | **Taste** | **Degree of Acceptance** |
|---|---|---|---|
| 1 | 3 | 2 | 6 |
| 2 | 3 | 3 | 9 |
| 3 | 3 | 1 | 3 |
| 4 | 3 | 4 | 12 |
| 5 | 4 | 4 | 16 |
| 6 | 5 | 4 | 20 |
| 7 | 5 | 5 | 25 |
| 8 | 5 | 5 | 25 |
| 9 | 5 | 5 | 25 |
| 10 | 5 | 5 | 25 |
| 11 | 3 | 2 | 6 |
| 12 | 3 | 3 | 9 |
| 13 | 3 | 2 | 6 |
| 14 | 4 | 4 | 16 |
| 15 | 4 | 4 | 16 |
| 16 | 5 | 3 | 15 |
| 17 | 5 | 5 | 25 |
| 18 | 5 | 5 | 25 |
| 19 | 5 | 5 | 25 |
| 20 | 5 | 5 | 25 |
| 21 | 5 | 5 | 25 |
| 22 | 5 | 5 | 25 |
| 23 | 5 | 5 | 25 |
| 24 | 5 | 5 | 25 |
| 25 | 5 | 5 | 25 |

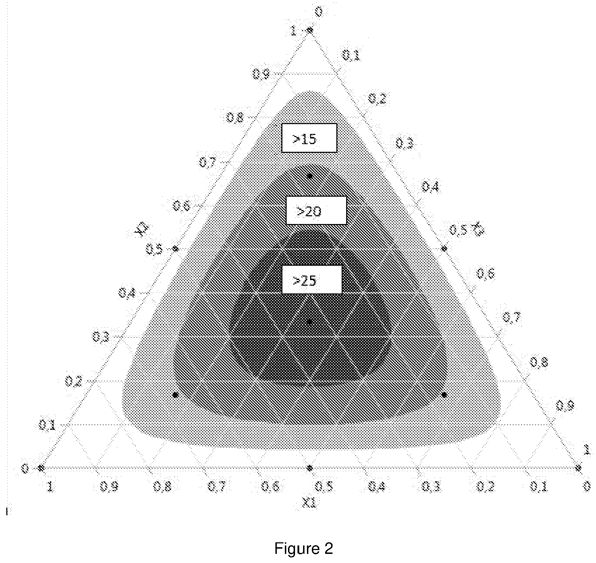

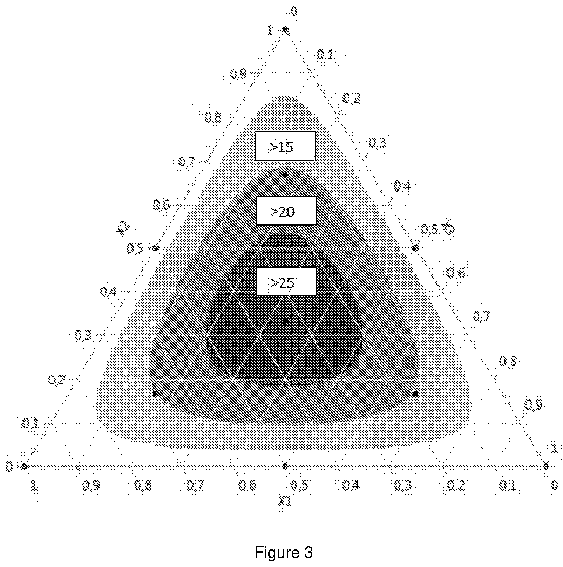

Figures 2 and 3 represent the isolines of the degree of acceptance of the orodispersible powders, prepared according to a mixture design, and comprising bilastine and levocetirizine respectively, as function of the composition of the synergistic ternary combination.

It can be observed that the ternary combinations provide a synergistic effect regarding the acceptance of the formulation by the patient. Further, the special cubic model described in a statistically significant way the acceptance behaviour of the mixtures.

The dissolution rates in the buccal cavity of the orodispersible powders dosed in stick packs, prepared according to Examples 7, 17 and 21 to 25, were carried out in 5 healthy volunteers. The results indicated that the formulations show a very rapid dissolution (about 10 seconds) in the buccal cavity, without leaving any residue of product in the mouth, and they were well accepted by the panel of testers.

### b) Physical parameters

The following galenic parameters were determined in order to assess the flowability of the formulations: angle of repose (degrees), compressibility index (%) and Hausner ratio. The methodology of the European Pharmacopoeia current edition was used, as shown in Table XI:

**TABLE XI**

| **Compressibility index** | **Hausner ratio** | **Angle of repose** | **Assessment** |
|---|---|---|---|
| 1-10 | 1.00-1.11 | 25-30 | Excellent |
| 11-15 | 1.12-1.18 | 31-35 | Good |
| 16-20 | 1.19-1.25 | 36-40 | Fair |
| 21-25 | 1.26-1.34 | 41-45 | Passable |
| 26-31 | 1.35-1.45 | 46-55 | Poor |
| 32-37 | 1.46-1.59 | 56-65 | Very poor |
| >38 | >1.60 | >66 | Very, very poor |

Table XII shows the results for several orodispersible powders according to the invention:

**TABLE XII**

| **Parameter** | **Ex. 21** | **Ex. 22** | **Ex. 23** | **Ex. 24** | **Ex. 25** | **Ex. 7** | **Ex. 17** |
|---|---|---|---|---|---|---|---|
| Compressibility index | 9 | 8 | 9 | 8 | 8 | 7 | 6 |
| Hausner ratio | 1.10 | 1.09 | 1.10 | 1.09 | 1.09 | 1.07 | 1.06 |
| Angle of repose | 28 | 29 | 28 | 29 | 28 | 27 | 27 |

The results indicate that the formulations have optimal flow properties, which were maintained even after 6 months of storage at room temperature.

## Claims

1. An orodispersible powder composition, **characterized in that** it comprises:
a) a pharmacologically effective amount of an antihistamine active compound or a pharmaceutically acceptable salt thereof,
b) a ternary combination consisting of sodium citrate, citric acid and sucralose, and
c) one or more further excipients,
wherein the ratio a):b) is comprised from 1:2 to 1:75,
wherein the ternary composition comprises 55% to 75% by weight of sodium citrate, 15% to 35% by weight of citric acid and 10% to 30% by weight of sucralose, wherein the amounts of sodium citrate, citric acid and sucralose in the ternary composition are combined to make 100%, and
wherein the content of the ternary combination in the orodispersible powder composition is comprised between 5% and 20% by weight over the total weight of the orodispersible powder composition.

2. The orodispersible powder composition according to claim 1, **characterized in that** the antihistamine active compound is selected from acrivastine, azatadine, azelastine, bilastine, brompheniramine, carbinoxamine, cetirizine, chlorpheniramine, clemastine, desloratadine, dexbropheniramine, dexchlorpheniramine, diphenylhydramine, diphenylpyraline, doxylamine, ebastine, fexofenadine, levocetirizine, loratadine, olopatadine, promethazine, pyrilamine, rupatadine, tripelennamine, triprolidine, or a salt thereof, and mixtures thereof.

3. The orodispersible powder composition according to claim 1 or 2, **characterized in that** the ratio antihistamine active compound:ternary combination is selected from between 1:5 and 1:75, preferably between 1:6 and 1:70, preferably between 1:7 and 1:65, and more preferably between 1:12.5 and 1:60.

4. The orodispersible powder composition according to any one of claims 1 to 3, **characterized in that** one or more further excipients are selected from diluents, lubricants, glidants, flavouring agents, sweetening agents, acidulants, alkalizing agents, buffering agents, antioxidants and mixtures thereof.

5. The orodispersible powder composition according to claim 4, **characterized in that** the diluent is selected from mannitol, sorbitol, xylitol, isomalt, erythritol, cellulose powdered, microcrystalline cellulose, silified microcrystalline cellulose, starch, calcium phosphate, calcium lactate, calcium carbonate, magnesium carbonate, magnesium oxide and mixtures thereof.

6. The orodispersible powder composition according to claim 4, **characterized in that** the lubricant is selected from the group consisting of talc, sodium benzoate, sodium stearyl fumarate, sodium lauryl sulfate, calcium stearate, magnesium stearate, zinc stearate, glyceryl behenate, stearic acid, glyceryl monostearate, poloxamer, polyethylene glycol and mixtures thereof.

7. The orodispersible powder composition according to claim 4, **characterized in that** the glidant is selected from the group consisting of colloidal silicon dioxide, calcium phosphate tribasic, hydrophobic colloidal silica, magnesium silicate, magnesium trisilicate, silicon dioxide, talc and mixtures thereof.

8. The orodispersible powder composition according to claim 4, **characterized in that** the flavouring agent is selected from orange, banana, strawberry, cherry, wild cherry, lemon, cardamom, anise, peppermint, menthol, vanillin and ethyl vanillin and mixtures thereof.

9. A stick pack comprising the orodispersible powder according to any one of claims 1 to 8.

10. A tablet comprising the orodispersible composition according to any one of claims 1 to 8.

11. The orodispersible powder composition according to any one of claims 1 to 8 and the stick pack of claim 9 and the tablet of claim 10 for use in the treatment of allergic diseases.

12. The orodispersible powder composition and the stick pack and the tablet according to claim 11 for use in the treatment of an allergic disease selected from seasonal allergic rhinitis, persistent allergic rhinitis, seasonal allergic rhino-conjunctivitis, perennial allergic rhino-conjunctivitis, urticaria, chronic idiopathic urticaria, and allergic dermatitis.

## Patentansprüche

1. Schmelzpulverzusammensetzung, **dadurch gekennzeichnet, dass** sie Folgendes umfasst:
a) eine pharmakologisch wirksame Menge eines Antihistamin-Wirkstoffs oder eines pharmazeutisch annehmbaren Salzes davon,
b) eine ternäre Kombination bestehend aus Natriumcitrat, Zitronensäure und Sucralose, und
c) einen oder mehrere weitere Hilfsstoffe,
wobei das Verhältnis a):b) zwischen 1:2 und 1:75 liegt,
wobei die ternäre Zusammensetzung 55 bis 75 Gew.-% Natriumcitrat, 15 bis 35 Gew.-% Zitronensäure und 10 bis 30 Gew.-% Sucralose umfasst, wobei die Mengen an Natriumcitrat, Zitronensäure und Sucralose in der ternären Zusammensetzung kombiniert werden, um 100 % zu ergeben, und
wobei der Gehalt der ternären Kombination in der Schmelzpulverzusammensetzung zwischen 5 und 20 Gew.-%, bezogen auf das Gesamtgewicht der Schmelzpulverzusammensetzung, beträgt.

2. Schmelzpulverzusammensetzung nach Anspruch 1, **dadurch gekennzeichnet, dass** der Antihistamin-Wirkstoff ausgewählt ist aus Acrivastin, Azatadin, Azelastin, Bilastin, Brompheniramin, Carbinoxamin, Cetirizin, Chlorpheniramin, Clemastin, Desloratadin, Dexbropheniramin, Dexchlorpheniramin, Diphenylhydramin, Diphenylpyralin, Doxylamin, Ebastin, Fexofenadin, Levocetirizin, Loratadin, Olopatadin, Promethazin, Pyrilamin, Rupatadin, Tripelennamin, Triprolidin oder ein Salz davon sowie Mischungen davon.

3. Schmelzpulverzusammensetzung nach Anspruch 1 oder 2, **dadurch gekennzeichnet, dass** das Verhältnis Antihistamin-Wirkstoff:ternäre Kombination zwischen 1:5 und 1:75, vorzugsweise zwischen 1:6 und 1:70, vorzugsweise zwischen 1:7 und 1:65 und besonders bevorzugt zwischen 1:12,5 und 1:60 ausgewählt ist.

4. Schmelzpulverzusammensetzung nach einem der Ansprüche 1 bis 3, **dadurch gekennzeichnet, dass** ein oder mehrere weitere Hilfsstoffe ausgewählt sind aus Verdünnungsmitteln, Gleitmitteln, Gleitstoffen, Aromastoffen, Süßungsmitteln, Säuerungsmitteln, Alkalisierungsmitteln, Puffermitteln, Antioxidantien und Mischungen davon.

5. Schmelzpulverzusammensetzung nach Anspruch 4, **dadurch gekennzeichnet, dass** das Verdünnungsmittel ausgewählt ist aus Mannitol, Sorbitol, Xylitol, Isomalt, Erythritol, Zellulosepulver, mikrokristalliner Zellulose, silifizierter mikrokristalliner Zellulose, Stärke, Calciumphosphat, Calciumlactat, Calciumcarbonat, Magnesiumkarbonat, Magnesiumoxid und Mischungen davon.

6. Schmelzpulverzusammensetzung nach Anspruch 4, **dadurch gekennzeichnet, dass** das Gleitmittel ausgewählt ist aus der Gruppe bestehend aus Talkum, Natriumbenzoat, Natriumstearylfumarat, Natriumlaurylsulfat, Calciumstearat, Magnesiumstearat, Zinkstearat, Glycerylbehenat, Stearinsäure, Glycerylmonostearat, Poloxamer, Polyethylenglykol und Mischungen davon.

7. Schmelzpulverzusammensetzung nach Anspruch 4, **dadurch gekennzeichnet, dass** der Gleitstoff ausgewählt ist aus der Gruppe bestehend aus kolloidalem Siliciumdioxid, dreibasischem Calciumphosphat, hydrophobem kolloidalem Siliciumdioxid, Magnesiumsilicat, Magnesiumtrisilicat, Siliciumdioxid, Talkum und Mischungen davon.

8. Schmelzpulverzusammensetzung nach Anspruch 4, **dadurch gekennzeichnet, dass** der Aromastoff ausgewählt ist aus Orange, Banane, Erdbeere, Kirsche, Wildkirsche, Zitrone, Kardamom, Anis, Pfefferminze, Menthol, Vanillin und Ethylvanillin und Mischungen davon.

9. Stickpack, das das Schmelzpulver nach einem der Ansprüche 1 bis 8 umfasst.

10. Tablette, die die Schmelzzusammensetzung nach einem der Ansprüche 1 bis 8 umfasst.

11. Schmelzpulverzusammensetzung nach einem der Ansprüche 1 bis 8 und Stickpack nach Anspruch 9 und Tablette nach Anspruch 10 zur Verwendung bei der Behandlung von allergischen Erkrankungen.

12. Schmelzpulverzusammensetzung und Stickpack und Tablette nach Anspruch 11 zur Verwendung bei der Behandlung einer allergischen Erkrankung, ausgewählt aus saisonaler allergischer Rhinitis, persistierender allergischer Rhinitis, saisonaler allergischer Rhinokonjunktivitis, ganzjähriger allergischer Rhinokonjunktivitis, Urtikaria, chronischer idiopathischer Urtikaria und allergischer Dermatitis.

## Revendications

1. Composition de poudre orodispersible, **caractérisée en ce qu'**elle comprend :
a) une quantité pharmacologiquement efficace d'un composé actif antihistaminique ou d'un sel pharmaceutiquement acceptable de celui-ci,
b) une combinaison ternaire constituée de citrate de sodium, d'acide citrique et de sucralose et
c) un ou plusieurs autres excipients,
dans laquelle le rapport a):b) est compris entre 1:2 et 1:75,
dans laquelle la composition ternaire comprend de 55 % à 75 % en poids de citrate de sodium, 15 % à 35 % en poids d'acide citrique et 10 % à 30 % en poids de sucralose, dans laquelle les quantités de citrate de sodium, l'acide citrique et le sucralose dans la composition ternaire sont combinés pour obtenir 100 %, et
dans laquelle la teneur de la combinaison ternaire dans la composition de poudre orodispersible est comprise entre 5 % et 20 % en poids par rapport au poids total de la composition de poudre orodispersible.

2. Composition de poudre orodispersible selon la revendication 1, **caractérisée en ce que** le composé actif antihistaminique est choisi parmi l'acrivastine, l'azatadine, l'azélastine, la bilastine, la bromphéniramine, la carbinoxamine, la cétirizine, la chlorphéniramine, la clémastine, la desloratadine, la dexbrophéniramine, la dexchlorphéniramine, la diphénylhydramine, la diphénylpyraline, la doxylamine, l'ébastine, la fexofénadine, la lévocétirizine, la loratadine, l'olopatadine, la prométhazine, la pyrilamine, la rupatadine, la tripelennamine, la triprolidine, ou un de ses sels, et leurs mélanges.

3. Composition de poudre orodispersible selon la revendication 1 ou 2, **caractérisée en ce que** le rapport composé actif antihistaminique/combinaison ternaire est compris entre 1:5 et 1:75, de préférence entre 1:6 et 1:70, de préférence entre 1:7 et 1:65, et plus préférentiellement entre 1:12,5 et 1:60.

4. Composition de poudre orodispersible selon l'une quelconque des revendications 1 à 3, **caractérisée en ce qu'**un ou plusieurs autres excipients sont choisis parmi des diluants, des lubrifiants, des agents de glissement, des agents aromatisants, des agents édulcorants, des acidulants, des agents alcalinisants, des agents tampons, des antioxydants et des mélanges de ceux-ci.

5. Composition de poudre orodispersible selon la revendication 4, **caractérisée en ce que** le diluant est choisi parmi le mannitol, le sorbitol, le xylitol, l'isomalt, l'érythritol, la cellulose en poudre, la cellulose microcristalline, la cellulose microcristalline silicifiée, l'amidon, le phosphate de calcium, le lactate de calcium, le carbonate de calcium, le carbonate de magnésium, l'oxyde de magnésium et des mélanges de ceux-ci.

6. Composition de poudre orodispersible selon la revendication 4, **caractérisée en ce que** le lubrifiant est choisi dans le groupe constitué par le talc, le benzoate de sodium, le stéarylfumarate de sodium, le laurylsulfate de sodium, le stéarate de calcium, le stéarate de magnésium, le stéarate de zinc, le béhénate de glycéryle, l'acide stéarique, le monostéarate de glycéryle, un poloxamère, le polyéthylèneglycol et les mélanges de ceux-ci.

7. Composition de poudre orodispersible selon la revendication 4, **caractérisée en ce que** l'agent de glissement est choisi dans le groupe constitué par le dioxyde de silicium colloïdal, le phosphate tricalcique, la silice colloïdale hydrophobe, le silicate de magnésium, le trisilicate de magnésium, le dioxyde de silicium, le talc et les mélanges de ceux-ci.

8. Composition de poudre orodispersible selon la revendication 4, **caractérisée en ce que** l'agent aromatisant est choisi parmi l'orange, la banane, la fraise, la cerise, la cerise douce, le citron, la cardamome, l'anis, la menthe poivrée, le menthol, la vanilline et l'éthylvanilline et des mélanges de ceux-ci.

9. Emballage sous forme de stick comprenant la poudre orodispersible selon l'une quelconque des revendications 1 à 8.

10. Comprimé comprenant la composition orodispersible selon l'une des revendications 1 à 8.

11. Composition de poudre orodispersible selon l'une des revendications 1 à 8, le stick pack de la revendication 9 et le comprimé de la revendication 10 pour une utilisation dans le traitement des maladies allergiques.

12. Composition de poudre orodispersible, le stick pack et le comprimé selon la revendication 11 pour le traitement d'une maladie allergique choisie parmi la rhinite allergique saisonnière, la rhinite allergique persistante, la rhino-conjonctivite allergique saisonnière, la rhino-conjonctivite allergique perannuelle, l'urticaire, l'urticaire chronique idiopathique et la dermatite allergique.
